# EUROPEAN PATENT APPLICATION

(11) **EP 4 574 814 A1**
(43) Date of publication of application: **25.06.2025**
(21) Application number: 24219468.6
(22) Date of filing: 12.12.2024
(51) Int. Cl.: C07C 243/38, G02F 1/361, C07D 201/00, C09J 4/00, C07D 305/08, C09D 4/00, C09K 3/10

(54) **COMPOUND, CURABLE RESIN COMPOSITION, CURED PRODUCT, AND DECOMPOSITION METHOD**

(30) Priority: 19.12.2023 JP 2023214033
(71) Applicant: ThreeBond Co., Ltd., Tokyo 192-0398 (JP)
(72) Inventor: Furukawa, Masazumi, Hachioji-shi, Tokyo, 192-0398 (JP)
(74) Representative: Grünecker Patent- und Rechtsanwälte PartG mbB

(57) **Abstract**

A compound represented by a general formula (1) in which R¹ represents a divalent or higher organic group, R² represents a hydrogen atom or an alkyl group, R³ represents a monovalent organic group including a reactive functional group, and n represents an integer of 2 to 10. The reactive functional group in R³ may be at least one selected from the group consisting of a glycidyl group, a (meth)acryloyl group, an epoxycyclohexyl group, an oxetanyl group, an allyl group, and a vinyl group.

## Description

### CROSS-REFERENCE TO RELATED APPLICATIONS

The present application is based on Japanese Patent Application No. 2023-214033 filed on December 19, 2023, the contents thereof being hereby incorporated by reference.

### TECHNICAL FIELD

The present invention relates to a compound, a curable resin composition, a cured product, and a decomposition method. The present invention also relates to an adhesive, a sealing agent, and a coating agent.

### BACKGROUND ART

An adhesive is used to bond two or more different members, and a high adhesion strength is generally required. However, in recent years, from the viewpoint of reuse of resources, a dismantlable adhesive, which enables reuse of members by dismantling adhesive surfaces after use, has been required (Patent Literature 1). In the related art, there are various types of curing forms for an adhesive, such as heat curing and photo curing, and the curing form is selected depending on the members and conditions used.

### CITATION LIST

### PATENT LITERATURE

Patent Literature 1: WO2007/083566

### SUMMARY OF INVENTION

The dismantlable adhesives according to the related art are required to be synthesized in multiple stages, and large labor and cost are required for industrial production. Therefore, the curing form of the dismantlable adhesive according to the related art is limited to only certain curing forms.

As a result of diligent studies to solve the above problems, the present inventors have found a compound that can be easily synthesized and has decomposability against an oxidant. The present inventors have also found that a curable resin composition including the compound can form a cured product having excellent dismantlability.

The gist of the present invention will be described below.
1. A compound represented by the following general formula (1): 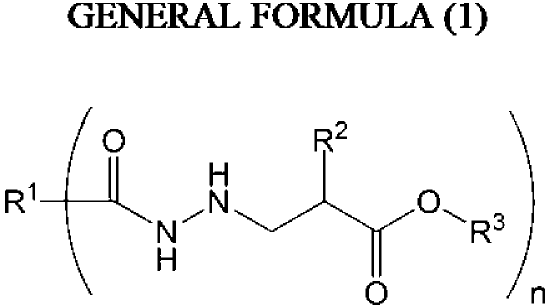 where R¹ represents a divalent or higher organic group, R² represents a hydrogen atom or an alkyl group, R³ represents a monovalent organic group including a reactive functional group, and n represents an integer of 2 to 10.
2. The compound according to the above item 1, in which the reactive functional group in R³ is at least one selected from the group consisting of a glycidyl group, a (meth)acryloyl group, an epoxycyclohexyl group, an oxetanyl group, an allyl group, and a vinyl group.
3. The compound according to the above item 1 or 2, in which R¹ represents a divalent or higher organic group including one or more aromatic rings.
4. A cured product obtained by curing the compound according to the above item 1 or 2 with at least one selected from the group consisting of heat curing, photo curing, moisture curing, and anaerobic curing.
5. A curable resin composition including the compound according to the above item 1 or 2.
6. An adhesive, a sealing agent or a coating agent including the compound according to the above item 1 or 2.
7. A cured product obtained by curing the adhesive, the sealing agent or the coating agent according to the above item 6 with at least one selected from the group consisting of heat curing, photo curing, moisture curing, and anaerobic curing.
8. A decomposition method including decomposing the cured product according to the above item 4 using an oxidant.
9. The decomposition method according to the above item 8, in which the oxidant is an aqueous hypochlorous acid solution.

The compound according to the present invention (hereinafter, may be referred to as "decomposable compound") can be easily synthesized. In addition, the decomposable compound and a cured product obtained from the decomposable compound can be decomposed by an oxidant, and dismantlability can be imparted to a laminated body prepared using an adhesive, a sealing agent, and a coating agent including the decomposable compound according to the present invention.

### DESCRIPTION OF EMBODIMENTS

The present invention will be described in detail below. In the present specification, "X to Y" means that numerical values (X and Y) described before and after "to" are included as a lower limit value and an upper limit value, and means "X or more and Y or less". In the present invention, a compound including a (meth)acryloyl group refers to (meth)acrylate. The (meth)acryloyl group may be in the form of a (meth)acryloyloxy group. The term "(meth)acryloyl" includes both acryloyl and methacryloyl. Accordingly, for example, the term "(meth)acryloyl group" includes both an acryloyl group (H₂C=CH-C(=O)-) and a methacryloyl group (H₂C=C(CH₃)-C(=O)-). Similarly, the term "(meth)acrylate" includes both acrylate and methacrylate, the term "(meth)acrylic" includes both acrylic and methacrylic, and the term "(meth)acrylamide" includes both acrylamide and methacrylamide.

The decomposable compound according to the present invention is represented by the following general formula (1).

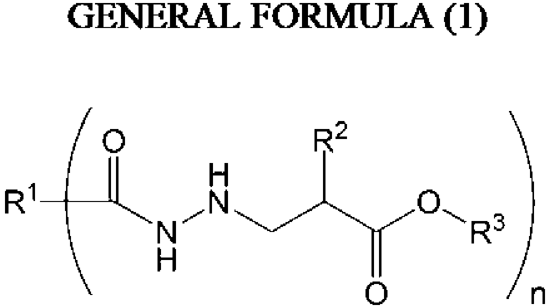

(In the general formula (1), R¹ represents a divalent or higher organic group, R² represents a hydrogen atom or an alkyl group, R³ represents a monovalent organic group including a reactive functional group, and n represents an integer of 2 to 10.)

The decomposable compound according to the present invention exhibits excellent solubility (decomposability) in a solution including an oxidant.

The compound represented by the general formula (1) can be obtained by causing a reaction of a compound having a structure represented by the following general formula (2) and a compound having a structure represented by the following general formula (3) or a structure represented by the following general formula (4).

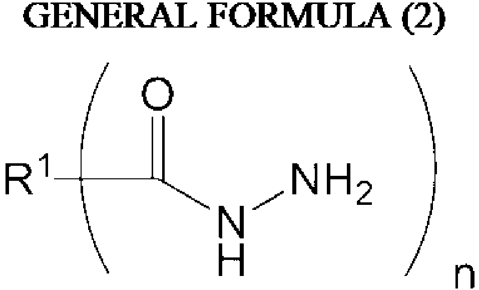

(In the general formula (2), R¹ represents a divalent or higher organic group, and n represents an integer of 2 to 10.)

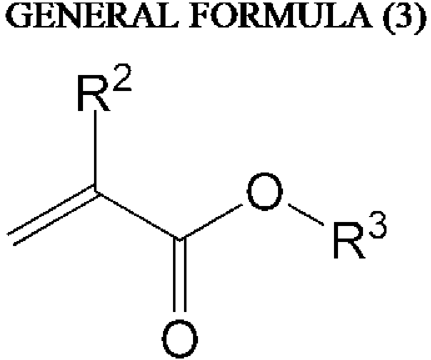

(In the general formula (3), R² represents a hydrogen atom or an alkyl group, and R³ represents a monovalent organic group including a reactive functional group.)

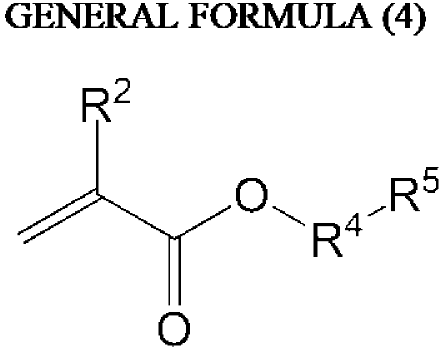

(In the general formula (4), R² represents a hydrogen atom or an alkyl group, R⁴ represents a divalent organic group, and R⁵ represents a reactive functional group.)

In the general formulas (1) and (2), R¹ preferably represents an aliphatic hydrocarbon group and/or an aromatic hydrocarbon group. Specific examples of the compound represented by the general formula (2) include aliphatic hydrocarbons such as adipic acid dihydrazide, sebacic acid dihydrazide, dodecanoic acid dihydrazide, azelaic acid dihydrazide, malonic acid dihydrazide, and 7,11-octadecadiene-1,18-dicarbohydrazide; aromatic hydrocarbons such as isophthalic acid dihydrazide and terephthalic acid dihydrazide; and alicyclic hydrocarbons such as 1,3-bis(hydrazinocarbonoethyl)-5-isopropylhydantoin. From the viewpoint of dismantlability during the reaction with the compound represented by the general formula (3), R¹ preferably represents a hydrocarbon group, R¹ more preferably includes an aromatic hydrocarbon group (that is, includes one or more aromatic rings), and the compound represented by the general formula (2) most preferably represents isophthalic acid dihydrazide.

The commercially available product of the compound represented by the general formula (2) is not particularly limited, and examples thereof include ADH, SDH, DDH, IDH, SAH (manufactured by Otsuka Chemical Co., Ltd.), AJICURE VDH, and UDH (manufactured by Ajinomoto Fine-Techno Co., Inc.).

Examples of the reactive functional groups in the general formula (1), the general formula (3), and the general formula (4) include a glycidyl group, a (meth)acryloyl group, an epoxycyclohexyl group, an oxetanyl group, an allyl group, and a vinyl group, but the reactive functional groups are not limited to these examples. Curing in various curing forms is enabled by selecting the reactive functional group.

Specific examples of the compound represented by the general formula (3) or (4) include a compound including two or more (meth)acryloyl groups, and a compound including one or more (meth)acryloyl groups and one or more reactive functional groups different from the (meth)acryloyl groups in one molecule, but the compound represented by the general formula (3) or (4) is not limited to these examples. Specific examples of the reactive functional group include a glycidyl group, an epoxycyclohexyl group, an oxetanyl group, an allyl group, and a vinyl group. By selecting the reactive functional group, various curing forms such as heat curing, photo curing, moisture curing, and anaerobic curing can be selected for the decomposable compound represented by the general formula (1) obtained by reaction with the compound represented by the general formula (2).

The compound including two or more (meth)acryloyl groups is preferably a compound including both an acryloyl group and a methacryloyl group.

The cured product according to the present invention is obtained by curing the compound according to the present invention with at least one selected from the group consisting of heat curing, photo curing, moisture curing, and anaerobic curing.

Specific examples of the compound including one or more (meth)acryloyl groups and including a glycidyl group as a reactive functional group include glycidyl (meth)acrylate and 4-hydroxybutyl acrylate glycidyl ether, but the compound is not limited to these examples.

Specific examples of the compound including one or more (meth)acryloyl groups and including an epoxycyclohexyl group as a reactive functional group include 3,4-epoxycyclohexylmethyl (meth)acrylate, but the compound is not limited to this example.

Specific examples of the compound including one or more (meth)acryloyl groups and including an oxetanyl group as a reactive functional group include (3-ethyloxetan-3-yl) (meth)acrylate, but the compound is not limited to this example.

Specific examples of the compound including one or more (meth)acryloyl groups and including an allyl group as a reactive functional group include 2-propenyl (meth)acrylate, but the compound is not limited to this example.

Specific examples of the compound including one or more (meth)acryloyl groups and including a vinyl group as a reactive functional group include vinyl (meth)acrylate and 2-[2-(vinyloxy)ethoxy]ethyl (meth)acrylate, but the compound is not limited to these examples.

It should be noted that the vinyl group includes a vinyl ether group (CH₂=CH-O-).

Examples of the compound including two or more (meth)acryloyl groups in one molecule include triethylene glycol di(meth)acrylate, polyethylene glycol #200 di(meth)acrylate, polyethylene glycol #400 di(meth)acrylate, polyethylene glycol #600 di(meth)acrylate, polyethylene glycol #1000 di(meth)acrylate, tripropylene glycol di(meth)acrylate, polypropylene glycol #400 di(meth)acrylate, polypropylene glycol #700 di(meth)acrylate, polytetramethylene glycol di(meth)acrylate, neopentyl glycol di(meth)acrylate, 3-methyl-1,5 pentanediol di(meth)acrylate, 1,6-hexanediol di(meth)acrylate, 1,9-nonanediol di(meth)acrylate, 1,10-decanediol di(meth)acrylate, dimethylol tricyclodecane di(meth)acrylate, di(meth)acrylate of bisphenol A-EO adduct, di(meth)acrylate of bisphenol A-PO adduct, hydroxypivalic acid-neopentyl glycol (meth)acrylic acid adduct, 2-hydroxy-3-acryloyloxypropyl methacrylate, 2-hydroxy-3-methacryl-propyl acrylate, trimethylolpropane tri(meth)acrylate, ethoxylated trimethylolpropane tri(meth)acrylate, ethoxylated glycerin tri(meth)acrylate, tris(2-acryloxyethyl)isocyanurate, bis(2-acryloxyethyl)isocyanurate, pentaerythritol tri(meth)acrylate, pentaerythritol tetra(meth)acrylate, ethoxylated pentaerythritol tetra(meth)acrylate, ditrimethylolpropane tetra(meth)acrylate, dipentaerythritol hexa(meth)acrylate, and ethoxylated dipentaerythritol poly(meth)acrylate. One of them may be used alone, or two or more thereof may be used in combination. Among them, from the viewpoint of achieving both the adhesion strength and the dismantlability, it is preferable to include at least one compound selected from the group consisting of trimethylolpropane tri(meth)acrylate, dimethylol-tricyclodecane di(meth)acrylate, and polyethylene glycol di(meth)acrylate, it is more preferable to include at least one compound selected from the group consisting of trimethylolpropane triacrylate, dimethylol-tricyclodecane diacrylate, polyethylene glycol diacrylate, and 2-hydroxy-3-methacrylpropyl acrylate, and it is most preferable to include 2-hydroxy-3-methacrylpropyl acrylate.

The commercially available product of the compound including two or more (meth)acryloyl groups in one molecule is not particularly limited, and examples thereof include LIGHT ACRYLATE 9EG-A, LIGHT ACRYLATE 14EG-A, TMP-A, and DCP-A (manufactured by KYOEI CHEMICAL CO., LTD.), NK ESTER A-200, A-400, A-600, A-1000, A-DCP, A-TMPT, and 701A (manufactured by SHIN NAKAMURA CHEMICAL CO., LTD.).

Specific examples of the divalent or higher organic group represented by R¹ include a divalent methylene group including 1 to 15 carbon atoms, a divalent aryl group, and a divalent organic group including an aromatic group.

Specific examples of the alkyl group represented by R² include a methyl group, an ethyl group, a propyl group, a butyl group, a pentyl group, a hexyl group, a heptyl group, and an octyl group.

The symbol n preferably represents 1 or 2.

Specific examples of the divalent organic group represented by R⁴ include a divalent methylene group, and the divalent methylene group preferably includes a hydroxy group or an ether group.

In the case of causing a reaction of the compound represented by the general formula (2) and the compound represented by the general formula (3) or (4), a functional group equivalent ratio (hydrazide group:(meth)acryloyl group) of the hydrazide group derived from the general formula (2) to the (meth)acryloyl group derived from the general formula (3) or (4) is preferably 1.0:1.0 to 1.0:3.0, more preferably 1.0:1.2 to 1.0:2.8, and most preferably 1.0:1.5 to 1.0:2.5. When the functional group equivalent ratio is 1.0:1.0 to 1.0:3.0, the yield of the decomposable compound can be improved.

The reaction can be performed by stirring in the presence of a solvent. Examples of the solvent include formamide, sulfolane, N,N-dimethylformamide, dimethyl sulfoxide, hexamethylphosphoramide, N-methyl-2-pyrrolidone, tetrahydrofuran, and dimethylacetamide, but the solvent is not limited to these examples.

The reaction temperature is not particularly limited as long as it is not a temperature at which the solvent volatilizes, and the compound according to the present invention can react at 100°C to 200°C.

The reaction time is not particularly limited as long as it is 30 minutes or longer, and is preferably 30 minutes to 24 hours.

### <Curable Resin Composition>

The reactive functional group derived from the reactive functional group in the general formula (3) or (4) is present in one molecule of the compound according to the present invention, and therefore, a curable resin composition can be obtained by including a curing agent capable of reacting with the reactive functional group. Further, the curable resin composition can be cured by various curing methods to obtain a cured product, and the cured product can be dismantled by an oxidant.

The curable resin composition includes the compound according to the present invention. The content of the compound according to the present invention in the curable resin composition is preferably 1 mass% to 90 mass%, more preferably 5 mass% to 60 mass%, and most preferably 10 mass% to 50 mass%.

In the case where the reactive functional group in the general formula (1) is at least one selected from the group consisting of a glycidyl group, an epoxycyclohexyl group, and an oxetanyl group, examples of the curing agent include a latent curing agent, an amine compound, an acid anhydride, an imidazole compound, a hydrazide compound, a thiol compound, a urea compound, dicyandiamide, a microcapsule type curing agent, a cationic initiator, and a photocationic initiator. One of them may be used alone, or two or more thereof may be used in combination.

In the case where the reactive functional group in the general formula (1) is a (meth)acryloyl group, examples of the curing agent include a photoradical initiator, a thermal radical initiator, and an anaerobic curable catalyst.

Specific examples of the compound including a (meth)acryloyl group as the reactive functional group include 2-hydroxy-3-methacrylpropyl acrylate. From the viewpoint of the yield of the decomposable compound, the compound represented by the general formula (3) or (4) preferably has both an acryloyl group and a methacryloyl group, and the functional group equivalent ratio of the acryloyl group to the methacryloyl group is preferably 3:7 to 7:3.

In the case where the reactive functional group in the general formula (1) is an allyl group, a radical initiator capable of generating radical species by cleavage caused by light and heat by the ene-thiol reaction can be used. At this time, a polythiol compound or the like can be used as the curing agent.

In the case where the reactive functional group in the general formula (1) is a vinyl group, a metal catalyst or the like can be used as the curing agent. Examples of the compound including a vinyl group as the reactive functional group include 2-[2-(vinyloxy)ethoxy]ethyl (meth)acrylate, but the compound is not limited to limited to this example.

When the decomposable compound according to the present invention is synthesized, it is preferable not to use an epoxy resin including no (meth)acryloyl group. The epoxy resin in the present specification is a compound including no (meth)acryloyl group and including one or more epoxy groups in one molecule. The use of an epoxy resin is not preferred because the epoxy group reacts with the compound represented by the general formula (2), and the cured product may not express dismantlability.

The curable resin composition according to the present invention includes the decomposable compound according to the present invention. A cured product can be obtained by curing the decomposable compound according to the present invention or the curable resin composition according to the present invention using a curing agent. The curable resin composition according to the present invention may include additives such as an organic filler, a colorant, a plasticizer, a silane coupling agent, a leveling agent, and a rheology control agent within a range not impairing the characteristics of the present invention.

The curable resin composition may include, in addition to the decomposable compound according to the present invention, a compound including at least one group selected from the group consisting of a glycidyl group, a (meth)acryloyl group, an epoxycyclohexyl group, an oxetanyl group, an allyl group, and a vinyl group to the extent that the curable resin composition does not lose properties thereof. A mass ratio of the decomposable compound according to the present invention to the compound including at least one group selected from the group consisting of a glycidyl group, a (meth)acryloyl group, an epoxycyclohexyl group, an oxetanyl group, an allyl group, and a vinyl group is preferably 100:0 to 50:50, and most preferably 100:0.

### <Method for Curing Curable Resin Composition>

In a method for curing the curable resin composition, any conditions can be set in accordance with the curing form of the curable resin composition. For example, in the case of the heat-curable resin composition, the curing is preferably performed at 50°C to 300°C, and more preferably at 70°C to 200°C. The curing time in the case of heat curing is preferably 0.1 minutes to 200 minutes, and more preferably 1 minute to 100 minutes. In the case of an active energy ray-curable resin composition, the light source is not particularly limited, and a low pressure mercury lamp, a medium pressure mercury lamp, a high pressure mercury lamp, an ultra-high pressure mercury lamp, a black light lamp, a microwave excited mercury lamp, a metal halide lamp, a sodium lamp, a halogen lamp, a xenon lamp, an LED, a fluorescent lamp, sunlight, an electron beam irradiation device, and the like can be used. The amount of irradiation is preferably 5 to 50 kJ/m², and more preferably 7 to 40 kJ/m². In the case of the moisture-curable resin composition, the curing is preferably performed at 10°C to 50°C, and the humidity is preferably 30% to 80%. The curing time in the case of moisture curing is preferably 1 day to 2 weeks, and more preferably 3 days to 1 week. The anaerobic curable resin composition can be cured by blocking air (oxygen) and bringing it into contact with metal ions.

### <Decomposition Method>

A decomposition method according to the present invention includes a step of decomposing a cured product using an oxidant.

The cured product including the decomposable compound according to the present invention can be decomposed and/or dismantled more easily by an oxidant. Examples of the oxidant that can be used in the decomposition method according to the present invention include chlorine, bromine, hydrogen peroxide, and hypochlorous acid or a salt thereof. From the viewpoint of versatility, it is preferable to use an aqueous solution of a hypochlorite, and it is more preferable to use an aqueous solution of sodium hypochlorite. The concentration of the aqueous solution of sodium hypochlorite is preferably 1 mass% to 10 mass%.

### <Decomposition Conditions>

The decomposition temperature of the decomposable compound in the decomposition method according to the present invention and the cured product obtained by curing the decomposable compound is preferably 5°C to 70°C, more preferably 10°C to 60°C, and most preferably 20°C to 50°C from the viewpoint of safety. The contact time with the oxidant required for decomposition is not particularly limited, and is preferably 10 seconds to 24 hours, more preferably 1 minute to 24 hours, and most preferably 30 minutes to 20 hours.

### <Adherend>

The type of adherend to which the curable resin composition according to the present invention can be applied is not particularly limited, and examples thereof include metal, plastic, and rubber. Examples of the metal include iron, aluminum, SUS, nickel, zinc, magnesium, gold, silver, copper, and titanium. Examples of the plastic include a fiber-reinforced plastic (FRP), a glass fiber-reinforced plastic (GFRP), a carbon fiber-reinforced plastic (CFRP), polyacryl, polyester, polyamide, an acrylonitrile-butadiene-styrene copolymer (ABS), nylon 6, nylon 6,6, polycarbonate, polyacetal, polyethylene terephthalate, polybutylene terephthalate (PBT), polyphenylene sulfide, polyphenylene ether, polyether ether ketone, polyethylene, and polypropylene. Examples of the rubber include a nitrile rubber, a urethane rubber, a silicone rubber, and EPDM. Among them, dismantling is generally difficult in the case of metal because the metal tends to exhibit adhesion strength, but dismantling is easy when the use of the curable resin composition according to the present invention is used, and thus metal is suitable.

### <Applications>

The compound and the curable resin composition according to the present invention can be included in an adhesive, a sealing agent, and a coating agent and can be applied to various applications. A cured product can be obtained by curing an adhesive, a sealing agent, and a coating agent with at least one selected from the group consisting of heat curing, photo curing, moisture curing, and anaerobic curing. Examples of specific applications to which the compound and the curable resin composition according to the present invention can be applied include adhesion, sealing, casting, and coating of a switch portion for an automatic vehicle, a headlamp, an internal component of an engine, an electrical component, a drive engine, a brake oil tank, a front hood, a fender, a body panel of a door, a window, and the like; adhesion, sealing, casting, and coating of a flat panel display (a liquid crystal display, an organic EL display, a light-emitting diode display device, a field emission display), a video disc, a CD, a DVD, an MD, a pickup lens, a hard disk, and the like in the electronic materials field; adhesion, sealing, and coating of a lithium battery, a lithium-ion battery, a manganese battery, an alkaline battery, a fuel battery, a silicon-based solar battery, a dye-sensitized battery, an organic solar cell, and the like in the battery field; adhesion, sealing, and coating of optical fiber materials for a periphery of an optical switch and a periphery of an optical connector, an optical passive component, an optical circuit component, a periphery of an optoelectronic integrated circuit, and the like in the optical component field; adhesion, sealing, and coating of a camera module, a lens material, a viewfinder prism, a target prism, a viewfinder cover, a light receiving sensor, a photographic lens, a projection lens for a projection television in the optical equipment field; adhesives, lining materials, sealing materials, and coating materials for gas pipes, water pipes, and the like in the infrastructure field.

### EXAMPLES

Next, the present invention will be described in more detail with reference to Examples, but the present invention is not limited to these Examples.

Synthetic products 1 to 5 were prepared as decomposable compounds by the following method.

### [Example 1]

In a N,N-dimethylformamide (DMF) solvent, 10.8 g (1.1 eq) of isophthalic acid dihydrazide and 20.2 g (2.0 eq) of 4-hydrobutyl acrylate glycidyl ether were heated and stirred at 150°C for 1 hour. DMF was removed by distillation under reduced pressure to obtain a synthetic product 1.

### [Example 2]

In a DMF solvent, 10.3 g (1.1 eq) of isophthalic acid dihydrazide, 20.6 g (2.0 eq) of 2-hydroxy-3-methacrylpropyl acrylate, and 0.05 g (0.01 eq) of hydroquinone were heated and stirred at 150°C for 1 hour. DMF was removed by distillation under reduced pressure to obtain a synthetic product 2.

### [Example 3]

In a DMF solvent, 10.9 g (1.1 eq) of isophthalic acid dihydrazide and 20.1 g (2.0 eq) of 3,4-epoxycyclohexylmethyl methacrylate (CYCLOMER M100 manufactured by DAICEL-ALLNEX LTD.) were heated and stirred at 170°C for 1 hour. DMF was removed by distillation under reduced pressure to obtain a synthetic product 3.

### [Example 4]

In a DMF solvent, 72 g (1.1 eq) of isophthalic acid dihydrazide and 120 g (2.0 eq) of (3-ethyloxetane-3-yl)methacrylate (OXE-10 manufactured by TOAGOSEI CO., LTD.) were heated and stirred at 170°C for 1 hour. DMF was removed by distillation under reduced pressure to obtain a synthetic product 4.

### [Example 5]

In a DMF solvent, 20 g (1.1 eq) of isophthalic acid dihydrazide, 38.4 g (2.0 eq) of 2-[2-(vinyloxy)ethoxy]ethyl acrylate, and 0.1 g of hydroquinone were heated and stirred at 150°C for 1 hour. DMF was removed by distillation under reduced pressure to obtain a synthetic product 5.

### [Confirmation of Decomposability]

Each of the synthetic products 1 to 5 was weighed out in an amount of 1 g, immersed in warm water at 40°C or 6 mass% aqueous hypochlorous acid solution at 40°C for 24 hours, followed by filtering and drying, and then, the weight loss of the synthetic product was measured. The results are shown in Table 1.

**Table 1**

| | | Weight loss (g) |
|---|---|---|
| Synthetic product 1 | Warm water | -0.04 |
| | 6% aqueous hypochlorous acid solution | -0.12 |
| Synthetic product 2 | Warm water | -0.07 |
| | 6% aqueous hypochlorous acid solution | -0.17 |
| Synthetic product 3 | Warm water | -0.36 |
| | 6% aqueous hypochlorous acid solution | -0.49 |
| Synthetic product 4 | Warm water | -0.22 |
| | 6% aqueous hypochlorous acid solution | -0.42 |
| Synthetic product 5 | Warm water | 0 |
| | 6% aqueous hypochlorous acid solution | -0.34 |

As shown in Table 1, compared with the synthetic products 1 to 5 immersed in the warm water, a significant weight loss was confirmed in the synthetic products 1 to 5 immersed in the 6 mass% aqueous hypochlorous acid solution that is an oxidant. It can be seen from the result that the decomposable compounds in the synthetic products 1 to 5 have decomposability against the oxidant.

Further, curable resin compositions were prepared using the synthetic products 1 and 2.

### [Example 6]

The synthetic product 2 was sampled in an amount of 5 g, and 14 g of dimethylacrylamide and 0.4 g of diphenyl(2,4,6-trimethylbenzoyl)phosphine oxide were added thereto, followed by mixing by a mixer for 15 minutes in a light-shielding environment.

### [Example 7]

The synthetic product 4 was sampled in an amount of 5 g, and 2 g of 3-ethyl-3-hydroxymethyloxetane (trade name: OXT-101, manufactured by TOAGOSEI CO., LTD.), 3.5 g of a mixture of 4-methylhexahydrophthalic anhydride and hexahydrophthalic anhydride (7:3) (trade name: RIKACID MH700G, manufactured by NEW JAPAN CHEMICAL CO., LTD.), 0.1 g of potassium 2-ethylhexenate, 0.2 g of C15 crown ether, and 0.25 g of fumed silica (trade name: AEROSIL RY200, manufactured by NIPPON AEROSIL CO., LTD.) were added, followed by mixing by a mixer for 15 minutes.

### [Example 8]

The synthetic product 4 was sampled in an amount of 5 g, and 2 g of 3-ethyl-3-hydroxymethyloxetane (trade name: OXT-101, manufactured by TOAGOSEI CO., LTD.), 3.5 g of polyethylene glycol # 400 diglycidyl ether (trade name: EPOLIGHT 400E), 3.5 g of a urethane-modified epoxy resin (trade name: ADEKA RESIN EPU-73B, manufactured by ADEKA CORPORATION), 10.3 g of a mixture of 4-methylhexahydrophthalic anhydride and hexahydrophthalic anhydride (7:3) (trade name: RIKACID MH700G, manufactured by NEW JAPAN CHEMICAL CO., LTD.), 0.8 g of potassium 2-ethylhexenate, and 1.6 g of C15 crown ether were added thereto, followed mixing by a mixer for 15 minutes.

### [Comparative Example 1]

A composition was prepared in the same manner as in Example 6, except that 2-hydroxy-3-methacrylpropyl acrylate was used instead of the synthetic product 1.

### [Comparative Example 2]

Ten grams of a urethane-modified epoxy resin (trade name: ADEKA RESIN EPU-73B, manufactured by ADEKA CORPORATION), 6.7 g of a mixture of 4-methylhexahydrophthalic anhydride and hexahydrophthalic anhydride (7:3) (trade name: RIKACID MH700G, manufactured by NEW JAPAN CHEMICAL CO., LTD.), 0.2 g of potassium 2-ethylhexenate, and 0.4 g of C15 crown ether were added and mixed by a mixer for 15 minutes.

### [Confirmation of Dismantlability]

Each of the compositions of Example 6 and Comparative Example 1 was weighed out in an amount of 1 g, and a cured product was prepared with an ultraviolet conveyor belt irradiation device at an integrated light amount of 30 kJ/m². The prepared cured product was immersed in 400 mL of a 6 mass% mass aqueous hypochlorous acid solution at 50°C or warm water at 50°C in a 500 mL beaker and allowed to stand for 12 hours, and the state of the cured product was observed. The results are shown in Table 2.

**Table 2**

| | Example 6 | Comparative Example 1 |
|---|---|---|
| After immersion with aqueous hypochlorous acid solution | Dissolved | Swelled |
| After immersion with warm water | Swelled | Swelled |

As shown in Table 2, regarding the cured product in Example 6, the cured product immersed in the oxidant (aqueous hypochlorous acid solution) was dissolved, and the cured product could not be visually confirmed. The cured product immersed in the warm water simply swelled, and the cured product was confirmed. On the other hand, the cured product in Comparative Example 1 simply swelled in either the aqueous hypochlorous acid solution or the warm water, and the cured product was confirmed. From the above, it can be seen that the cured product made of the composition in Example 6 can be dismantled by the oxidant, and the cured product made of the composition in Comparative Example 1 does not have dismantlability.

### [Measurement of Shear Adhesion Strength Change Rate]

Each of the curable resin compositions in Examples 7 and 8 and Comparative Example 2 was applied to an aluminum plate (A1050P) having a width of 25 mm, a length of 100 mm, and a thickness of 1 mm using a spacer so as to have a coating area of 25 mm × 10 mm and a coating thickness of 1 mm. The coating part was overlapped with another aluminum plate, followed by curing at 120°C for 1 hour using a hot air drying oven, and a shear adhesion strength test piece was obtained. The prepared shear adhesion strength test piece was immersed in a 500 mL beaker including 400 mL of a 6 mass% aqueous sodium hypochlorite solution (KITCHEN HAITER, manufactured by KAO CORPORATION), followed by standing in a thermostatic chamber at 50°C for 16 hours. The immersed test piece was taken out from the beaker and allowed to stand for 12 hours in a fume hood in an atmosphere of 25°C and 55% RH to dry the test piece. The dried test piece was used as a test piece after immersion.

The maximum strength (MPa) of the test piece before immersion and the test piece after immersion was measured using a tensile tester at a tensile speed of 50 mm/min. The shear adhesion strength change rate was calculated by the following equation. The results are shown in Table 3. Shear adhesion strength change rate = [(maximum strength of test piece after immersion - maximum strength of test piece before immersion)/maximum strength of test piece before immersion] × 100 (%)

The acceptable criterion for dismantlability based on the shear adhesion strength change rate is -90% or less, and preferably -100%.

**Table 3**

| | Example 7 | Example 8 | Comparative Example 2 |
|---|---|---|---|
| Shear adhesion strength change rate | -100% | -100% | -43% |

From Table 3, the test pieces prepared by using each of the curable resin compositions in Examples 7 and 8 were detached by being immersed in an oxidant (aqueous hypochlorous acid solution) without applying force, and could be easily dismantled. On the other hand, in the test piece prepared by using the curable resin composition in Comparative Example 2, a decrease in strength was observed, but detachment of the test piece was not confirmed. From the above, it can be seen that the cured products made of the curable resin compositions in Examples 7 and 8 have dismantlability, and the cured product made of the curable resin composition in Comparative Example 2 does not have dismantlability.

Although the present invention has been described in detail with reference to specific embodiments, it is apparent to those skilled in the art that various changes and modifications can be made without departing from the spirit and scope of the present invention. The present application is based on a Japanese Patent Application (Japanese Patent Application No. 2023-214033) filed on December 19, 2023, the contents of which are incorporated herein by reference.

### INDUSTRIAL APPLICABILITY

The decomposable compound according to the present invention can be easily synthesized, and the decomposable compound and a cured product obtained from the decomposable compound can be more easily decomposed by an oxidant. Curing under various conditions is enabled by selecting a reactive functional group in the decomposable compound. Therefore, the decomposable compound according to the present invention can be developed in various fields such as an adhesive, a sealing agent, and a coating agent, which are required to have dismantlability.

## Claims

1. A compound represented by the following general formula (1): 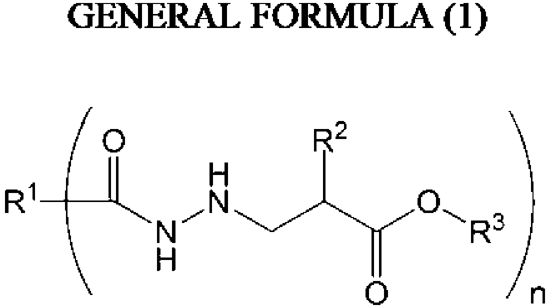 where R¹ represents a divalent or higher organic group, R² represents a hydrogen atom or an alkyl group, R³ represents a monovalent organic group comprising a reactive functional group, and n represents an integer of 2 to 10.

2. The compound according to claim 1, wherein the reactive functional group in R³ is at least one selected from the group consisting of a glycidyl group, a (meth)acryloyl group, an epoxycyclohexyl group, an oxetanyl group, an allyl group, and a vinyl group.

3. The compound according to claim 1 or 2, wherein R¹ represents a divalent or higher organic group comprising one or more aromatic rings.

4. A cured product obtained by curing the compound according to claim 1 or 2 with at least one selected from the group consisting of heat curing, photo curing, moisture curing, and anaerobic curing.

5. A curable resin composition comprising the compound according to claim 1 or 2.

6. An adhesive comprising the compound according to claim 1 or 2.

7. A sealing agent comprising the compound according to claim 1 or 2.

8. A coating agent comprising the compound according to claim 1 or 2.

9. A cured product obtained by curing the adhesive according to claim 6 with at least one selected from the group consisting of heat curing, photo curing, moisture curing, and anaerobic curing.

10. A cured product obtained by curing the sealing agent according to claim 7 with at least one selected from the group consisting of heat curing, photo curing, moisture curing, and anaerobic curing.

11. A cured product obtained by curing the coating agent according to claim 8 with at least one selected from the group consisting of heat curing, photo curing, moisture curing, and anaerobic curing.

12. A decomposition method comprising decomposing the cured product according to claim 4 using an oxidant.

13. The decomposition method according to claim 12, wherein the oxidant is an aqueous hypochlorous acid solution.
